# EUROPEAN PATENT SPECIFICATION

(11) **EP 4 182 006 B1**
(45) Date of publication and mention of the grant of the patent: **04.03.2026**
(21) Application number: 21845408.0
(22) Date of filing: 20.07.2021
(51) Int. Cl.: A61N 1/04, A61B 5/00, A61N 1/05, A61B 5/291, A61B 5/024

(54) **MODULAR AURICULAR SENSING SYSTEM**
MODULARES AURIKULÄRES SENSORSYSTEM
SYSTÈME MODULAIRE DE DÉTECTION AURICULAIRE

(30) Priority: 20.07.2020 US 202063053802 P
(43) Date of publication of application: 24.05.2023
(73) Proprietor: Nextsense, Inc., Mountain View, CA 94040 (US)
(72) Inventor: MIROV, Russell, Mountain View, California 94040 (US); ROBERTSON, Nick, Mountain View, California 94040 (US)
(74) Representative: Studio Torta S.p.A.
(86) International application number: PCT/US2021/042383
(87) International publication number: WO 2022/020358

(56) References cited:
- EP-A1- 3 154 275
- KR-B1- 101 791 038
- KR-B1- 102 102 421
- US-A- 4 622 975
- US-A1- 2006 206 014
- US-A1- 2007 112 277
- US-A1- 2015 303 619
- US-A1- 2019 192 077
- US-A1- 2019 192 077
- US-A1- 2019 209 038
- US-A1- 2019 209 038
- US-A1- 2019 223 747
- US-B2- 10 462 552

## Description

### BACKGROUND

Wearable sensors have been used to detect bio-signals, such as electroencephalogram (EEG) signals, electrocardiography (ECG) signals, and the like. In many applications, it is desirable to record such bio-signals over several days to a few weeks. These signals can be used for medical purposes (e.g., diagnostic sensing and/or therapeutic stimulation) or non-medical purposes (e.g., brain control interface). In the past, caps with sensors have been worn to capture EEG signals. These caps can acquire input via multiple data channels. However, wearing a cap for an extended period can be cumbersome and uncomfortable (e.g., overnight while sleeping) or socially awkward (e.g., in some office settings or formal social gatherings). It can also be difficult to obtain high quality signals because thick hair or scar tissue can interfere with signal detection. In-ear sensors, for instance using custom-molded earpieces, have also been considered and overcome several of the shortcomings of sensor caps. Unfortunately, even a custom in-ear sensor assembly may have various challenges, including providing a stable arrangement that is both securely and comfortably maintained in the ear. Other challenges can include poor signal conductivity, partially or completely blocking ambient sounds, as well as costly and labor-intensive manufacturing techniques.

US20190192077A1 discloses a system and method for extracting and analyzing in-ear electrical signals. The system includes electrode tips configured to conform to the user's ear canals and arranged with sense, reference, and driven ground electrodes. A signal acquisition subsystem obtains voltage differentials between electrodes placed in opposite ears, enabling the measurement of EEG signals from within the ear canals.

US20190209038A1 provides an in-ear EEG device and associated brain-computer interface technology. The device has an enclosure with a processor, power input, analog output, and an over-ear support arm coupled to an earpiece. EEG data is collected by the earpiece and processed by the enclosed electronics, allowing for the generation of output data. This configuration enables EEG measurement and subsequent data analysis within a compact ear-worn system.

KR102102421B1 relates to an EEG measuring earphone designed to detect drowsiness during driving. The earphone includes measurement, reference, and ground electrodes arranged to maintain good contact with the user's skin, even under minimal pressure, and without causing discomfort. By integrating a wired or wireless communication module, EEG signals can be transmitted for analysis, facilitating real-time monitoring of a driver's drowsiness in long-distance driving scenarios.

KR101791038B1 describes an apparatus and method for using ear-wearable EEG equipment to measure EEG signals around a user's ear region. The device receives brain signals and, based on a relationship model between various brain regions, estimates EEG characteristics for regions not directly monitored by the electrodes. This approach allows for broader EEG analysis through a limited set of ear-worn electrodes, potentially simplifying EEG measurement.

There is therefore a substantial need for improved wearable sensors.

### BRIEF SUMMARY

The aforementioned aim are reached by a modular auricular sensing system assembly as claimed in the appended set of claims.

The present technology relates to modular auricular sensing systems which can be used for sensing, recording and/or processing physiological signals, such as biometrics (e.g., photoplethysmogram (PPG) sensing for, e.g., heart rate, blood oxygen and/or respiration rate monitoring). The sensing systems can be used for other types of bio signal detection, such as monitoring and recording EEG signals, ECG signals or the like, as well as a human-computer interface. The sensing system can include an in-ear earpiece module (e.g., a compliant plug element) configured to receive physiological signals and an electronics module (e.g., a sensor body) configured to contain electronics for recording and/or processing the physiological signals. The earpiece module has a housing and one or more electrodes with contacts along at least a portion of an exterior surface of the housing. The housing of the earpiece module has a first end portion and a second end portion opposite the first end portion, and the housing is configured for at least partial insertion into the ear canal along the first end portion. The second end portion is releasably coupled to the electronics module. For example, one of the earpiece module or electronics module may include a metal coupling plate while the other of the earpiece module or the electronics module may include a magnet to magnetically couple the earpiece module and the electronics module together. In some embodiments, the earpiece module has a metal coupling plate and the electronics module has a ring magnet. In some embodiments, the earpiece module has a magnet of one polarity and the electronics module has a magnet with the opposite polarity.

Such an arrangement enables relative movement such as rotation between the earpiece module and the electronics module while maintaining electrical connectivity between the electrode(s) carried by the earpiece module and the components of the electronics module. The earpiece module, for example, can have signal collection components (e.g., memory) and signal processing components (e.g., a microprocessor). The earpiece module and/or the electronics module may also include additional sensors.

The configuration of the first and second coupling elements enables for the quick exchange of earpiece modules, for instance to change the size of the earpiece module, to change the electrode configuration, for repair or cleaning, etc. It also enables swapping of electronics modules, which allows for selection of different electronics modules, recharging the battery, data downloading to an off-board processing system, etc.

According to aspects of the present technology, an auricular sensing system comprises an earpiece module (e.g., plug element) and an electronics module (e.g., a sensor body). The earpiece module has a housing, a first coupling element (e.g., a coupling plate), and at least one electrode. The housing includes a first end portion configured for at least partial insertion into an ear canal and a second end portion opposite the first end portion. The first coupling element can be a metal coupling plate secured to the second end portion of the housing. The electrodes are arranged along the housing between the first end portion and the second end portion. The electronics module has a second coupling element at a first side thereof, which can be a ring magnet configured to be magnetically coupled to the metal coupling plate defining the first coupling element. The first coupling element of the earpiece module is electrically coupled to the electrodes and configured to pass electrical signals from the electrodes to the electronics module via the second coupling element. In some embodiments, instead of transmitting the signals from the electrodes to the electronics module via the first and second coupling elements, the signals can be transmitted via first and second electrical contacts on the earpiece module and the electronics module, respectively, that are separate from the first and second coupling elements. For example, the earpiece module and electronics module can further include compression contacts that contact each other via the force applied by the first and second coupling elements.

In one example, the electrodes of the earpiece module are configured to detect bio-signals via the ear of the wearer. In another example, the second coupling element is a ring magnet and the first coupling element is a metal coupling plate configured to allow rotation and/or translation of the first coupling element relative to the second coupling element while maintaining electrical connectivity between the earpiece module and the electronics module.

The earpiece module has a through-hole (e.g., a channel or passageway) extending from the first end portion to the second end portion. The through-hole of the earpiece module may be arranged along a longitudinal axis of the earpiece module, and the first coupling element and the second coupling element are aligned along the longitudinal axis. In this case, the electronics module may include a speaker. The through-hole may be defined by a lumen of a stiffening tube extending along the longitudinal axis. The electronics module may also include a microphone acoustically coupled to the earpiece module via the through-hole of the earpiece module.

The first coupling element and the second coupling element may both be annular. The first coupling element may have a larger outer diameter than an outer diameter of the second coupling element. Additionally or alternatively, the first coupling element may have a larger inner diameter than an inner diameter of the second coupling element.

In some embodiments, the first coupling element is a coupling plate comprising a ferromagnetic material that is coated with an electrically conductive material. In some embodiments, the second coupling element is a ring magnet comprising Neodymium. The first coupling element may be washer-shaped and have a first side permanently secured to the second end portion of the housing. In this case, a second side of the first coupling element may be curved to mate with a contact surface of the second coupling element.

The electronics module may include a processor module operatively coupled to the second coupling element or another electrical contact to receive the electrical signals from the earpiece module and to perform on-board processing of the received electrical signals and/or to transmit the received electrical signals to a remote processing system. The electronics module may further contain one or more additional sensors, including at least one of a biosensor, an orientation sensor, or an accelerometer. By way of example, the additional sensors may include a temperature sensor.

In a further example, the sensing system further comprises a pair of interstitial flexible circuits compressed between the first coupling element and the second coupling element. The pair of interstitial flexible circuits can be configured to pass the electrical signals from the first coupling element to the second coupling element.

According to another aspect of the technology, a sensor system assembly is configured to detect and process bio-signals of a wearer. The sensor system assembly comprises the modular auricular sensing system as described above and a remote processing system. The remote processing system includes a transceiver configured for communication with a transceiver of the processor module and one or more processors configured to process the bio-signals received from the processor module.

According to a further aspect, a modular earpiece kit comprises a plurality of earpiece modules as described above, in which each of the plurality of earpiece modules have housings with at least one of a different housing size or a different configuration of electrodes. The kit can further include one or more electronics module as set forth above. For example, the kit can have a first electronics module with a different arrangement of sensor components than a second electronics module.

### BRIEF DESCRIPTION OF THE DRAWINGS

Figs. 1A-B illustrate an example human ear.
Figs. 2A-G illustrate an example of a modular auricular sensing system in accordance with aspects of the present technology.
Figs. 3A-C illustrate examples of electrode arrangements in accordance with aspects of the present technology.
Figs. 4A-C illustrate views of configurations of modular auricular sensing systems in accordance with aspects of the present technology.
Figs. 5A-C illustrate exploded views of modular auricular sensing systems in accordance with aspects of the present technology.
Figs. 6A-B illustrate a portion of modular auricular sensing systems in accordance with aspects of the present technology.
Fig. 7A illustrates examples of a first coupling element and a second coupling element in accordance with aspects of the present technology.
Figs. 7B and 7C are cross-sectional views of embodiments of first and second coupling elements in accordance with aspects of the present technology.
Fig. 8A illustrates modular auricular sensing assemblies in accordance with aspects of the present technology.
Fig. 8B illustrates an external processing system in accordance with aspects of the present technology.

### DETAILED DESCRIPTION

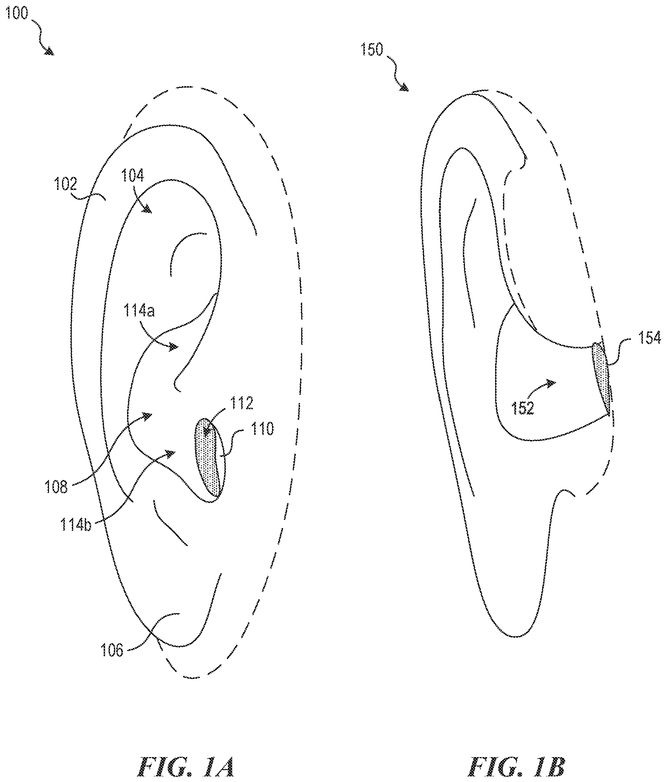

The human ear is a highly specialized organ that includes three main parts, the outer ear, middle ear and inner ear. An example of the outer ear (auricle or pinna) is illustrated in view 100 of Fig. 1A. As shown, the outer ear includes a helix 102, antihelix 104, lobule 106, concha 108, tragus 110 and opening to the ear canal 112. The concha 108 includes an upper region 114a known as cymba conchæ and a lower region 114b adjacent to the ear canal that is known as the cavum conchæ.

Fig. 1B illustrates another view 150 showing a partial cutaway perspective to illustrate the outer portion of the ear canal 152 leading to the ear drum (tympanic membrane) 154. The outer ear and ear canal are complex three-dimensional structures formed of flexible cartilage and skin. Thus, the shape of the outer ear can change when wearing hat or helmet, or when rolling over while laying down. For instance, the helix and antihelix may fold over, the lobule may be pulled away from or pushed toward the opening of the ear canal, and/or the tragus may be pushed inward towards the ear canal. As a result, an effective in-ear sensor system needs to be conformal, such as having a compliant 3D shape, while providing sufficient points of contact for the on-board sensors.

The present technology provides a modular auricular sensing system having an earpiece module releasably coupled to an electronics module (e.g., magnetically coupled). The magnetic coupling can also provide electrical connectivity between the earpiece module and electronics module and enable relative rotation and/or translation between the earpiece module and the electronics module during use in a user's ear. This arrangement provides for multiple degrees of freedom when the earpiece module and electronics module are connected, with one being able to rotate and/or slide relative to the other while maintaining electrical connectivity. The electronics module can be quickly disconnected from the earpiece module, for instance by pulling the electronics module away from the earpiece module.

The earpiece module includes a compliant housing configured to obtain bio-signals detected while in the ear canal. The earpiece module may have one or more electrodes arranged along the housing. For instance, the housing may comprise a foam, silicone or otherwise deformable material that can be compressed for insertion into the ear canal and then automatically expand to contact the ear canal at multiple points. During wear, the electronics module can be arranged along the concha. When not being worn, data stored by an on-board processing module can be downloaded to a remote, off-board processing system, for instance via a micro-USB connection or wireless link.

According to aspects of the present technology, one or more electrodes or other sensor elements are located along various points of the earpiece module, such as along an exterior surface of the earpiece module housing. Additional sensor elements may be disposed within or along the electronics module. Such an arrangement can be used for PPG sensing, other biometrics, a human-computer interface, or combination thereof.

By way of example only, embodiments of the sensing system may be beneficial in EEG, magnetoencephalography (MEG) or other diagnostic situations. For instance, Alpha waves on the order of 8-12 Hz can be detected either by either EEG or MEG. In addition, lower frequency signals (e.g., Delta waves between 0.5 - 3 Hz or Theta waves between 3 - 8 Hz) and/or higher frequency signals (e.g., Beta waves between 12 - 38 Hz or Gamma waves between 38-42 Hz) may also be detected. One or more of these types of signals can be evaluated and analyzed either alone or in conjunction with other data to provide information (e.g., biomarkers) about the wearer. The other data may be obtained by additional in-ear sensors (e.g., in the same earpiece module or in an earpiece module worn in the other ear) or sensors located elsewhere on or near the wearer. These additional sensors may include heart rate sensors, temperature sensors, and/or electrodermal activity (EDA) sensors that detect skin potential, resistance, conductance, admittance, or impedance, such as galvanic skin response sensors. Furthermore, still further additional sensors include pulse oximeter sensors, glucometers, orientation sensors, location sensors and/or accelerometers. All such sensors can be used in any combination. The biomarkers or other information can be evaluated to help classify mental or emotional states, as well as activities of daily living.

### EXAMPLE STRUCTURES

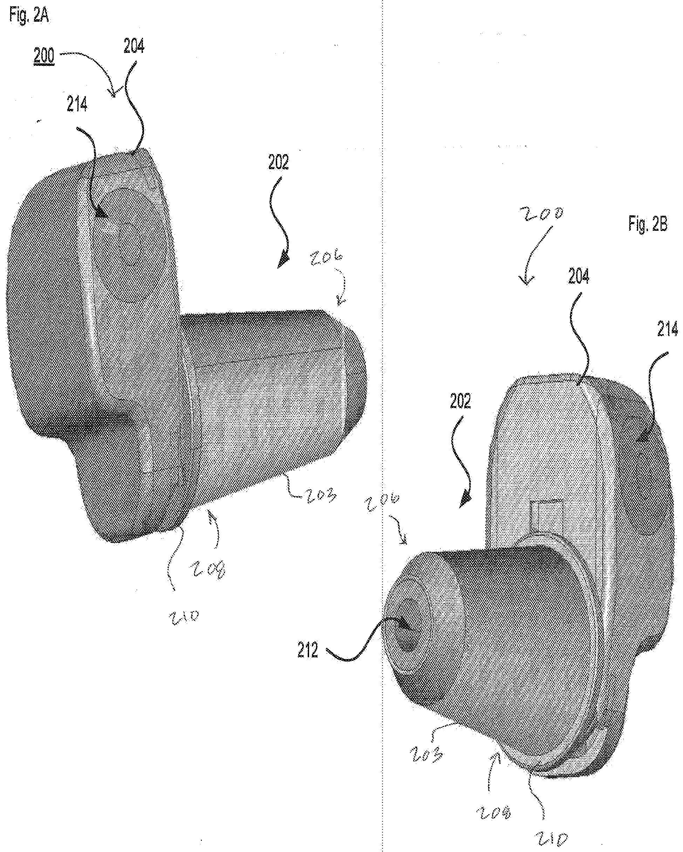

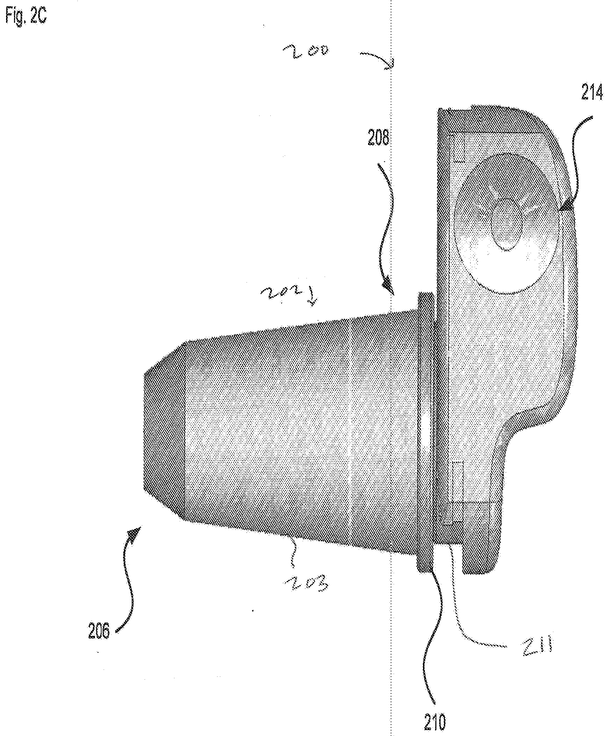

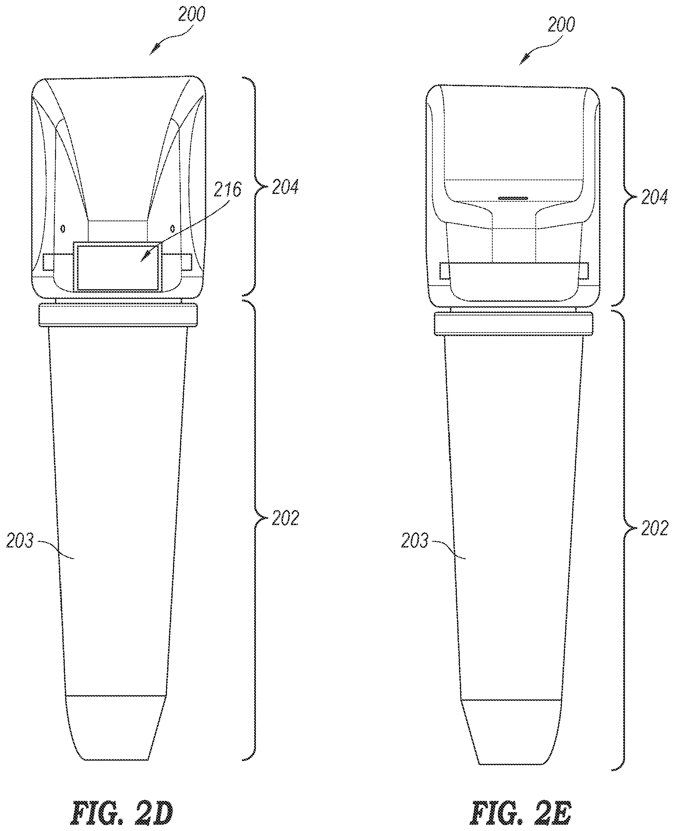

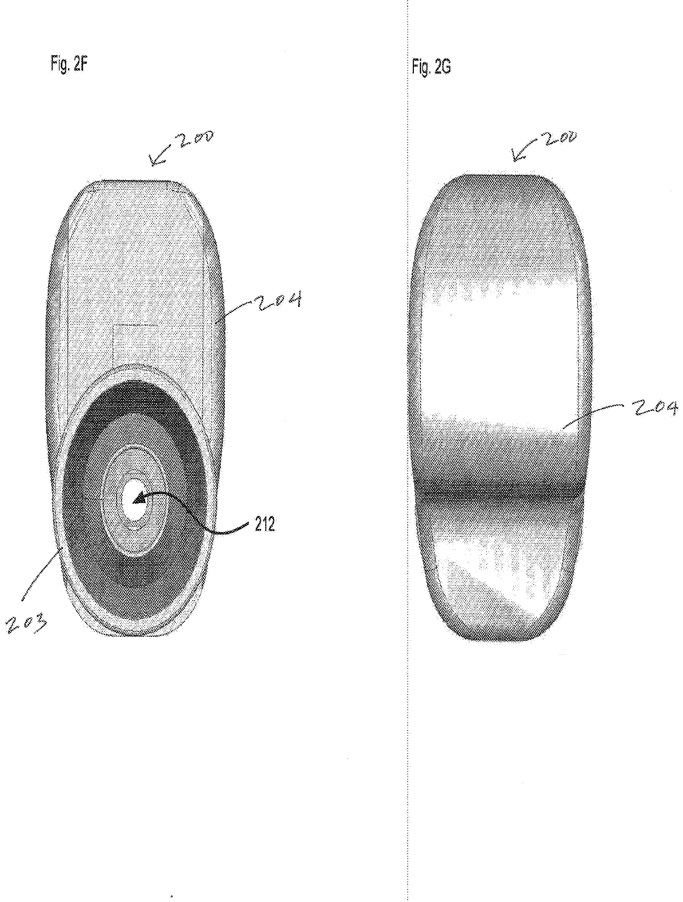

Figs. 2A-2G illustrate embodiments of modular auricular sensing systems 200 in accordance with aspects of the present technology. In some embodiments, the sensing system 200 includes an earpiece module 202 and an electronics module 204, as seen in the perspective views of Figs. 2A-B, the side view of Fig. 2C, the top view of Fig. 2D, the bottom view of Fig. 2E, the front view of Fig. 2F and the rear view of Fig. 2G. The earpiece module 202 has a housing 203 with a first end portion 206 and an opposing second end portion 208. The housing 203 can taper from a first diameter at first end portion 206 to a larger second diameter at the second end portion 208, and the first end portion 206 can be beveled to ease insertion into the ear canal of the user. The earpiece module 202 can also include a first coupling element 210 at the second end portion 208. In some embodiments, the first coupling element 210 is a metal plate (e.g., ferrite plate) or a magnet having a first polarity. In some embodiments, the first coupling element 210 may be bonded to the housing 203 using a conductive epoxy, silicone rubber or other suitable adhesive. In general, it may be desirable for the material adhering the first coupling element 210 to the housing 203 to be compliant to relieve stress between the first coupling element 210 and the housing 203 for structural integrity and comfort.

Referring to Figs. 2B and 2F, the housing 203 of the earpiece module 202 can have a through-hole 212 extending the length of the housing 203 from the first end portion 206 to the second end portion 208 defining a passageway or channel that provides an acoustic path between the electronics module 204 to the ear canal. While a single through-hole is shown, the housing 203 may have two or more through-holes, such as separate through-holes formed by one or more stiffening tubes extending through the housing 203. Additionally, the through-hole 212 may extend through only a portion of the housing 203, such as from the first end portion 206 to an intermediate distance short of the terminus of the second end portion 208.

At least an outer portion of the housing 203 of the earpiece module 202 may comprise a foam, silicone or other compliant material that can be compressed or otherwise deformed for insertion into the ear canal and optionally expand to at least generally conform to the topography of portions of the ear canal at multiple points after insertion. The exterior surface of the outer housing 203 may be coated with an electrically conductive material, such as a conductive polymer, to form one or more contacts (electrodes). Alternatively, the electrodes may be made from a bulk conductive material that is embedded within one or more regions of the housing 203 instead of just on the exterior surface of the housing 203. By way of example, the conductive polymer may include carbon particles (e.g., graphene) for conductivity and a silicone component. Other types of conductive polymers, such as Poly(3,4-ethylenedioxythiophene)-poly(styrenesulfonate) (PEDOT:PSS), may also be employed.

In both cases, the contact area(s) formed by the conductive material may be configured so that earpiece module 202 is orientation agnostic with respect to the anatomy of the ear canal. This enables the earpiece module 202 to have the desired signal connection to the neural and/or vascular structures in the ear without being limited to a specific rotational orientation relative to the ear canal. This can be particularly beneficial when the sensing system 200 is intended to be worn for several hours, days or even longer.

The electronics module 204 can be fabricated using different combinations and/or layers of soft, semi-soft, and hard materials of varying durometers for comfort and structural stability during use. For instance, conductive foam, silicone, nylon and other materials can be used at different locations along the exterior surfaces of the electronics module. In one scenario, harder acrylic-like materials could be coated with softer bio-compatible materials to enhance comfort for long-term wear. In one particular example, silicone is the primary bio-compatible material that may be over-molded on an acrylic base structure. In another scenario, conductive foam or the like may be used to enhance comfort to the wearer while also enhancing electrical contact along portions of the ear. Furthermore, the electronics module may be colored so that the device is unobtrusive and blends in with the wearer's ear.

The electronics module 204 can further include a second coupling element 211 configured to interface with the first coupling element 210 of the earpiece module 202 to releasably secure the electronics module 204 to the earpiece module 202. The second coupling element 211 can be a metal plate (e.g., ferrite plate) or a magnet having a second polarity. In some embodiments, the first coupling element 210 can be a metal plate and the second coupling element 211 can be a magnet configured to be magnetically attracted to the plate-type first coupling element 210. In some embodiments, the second coupling element 211 can be a metal plate and the first coupling element 210 can be a magnet configured to be magnetically attracted to the plate-type second coupling element 211. In some embodiments, the first coupling element 210 can be a magnet having a first polarity and the second coupling element 211 can be a magnet having a second polarity.

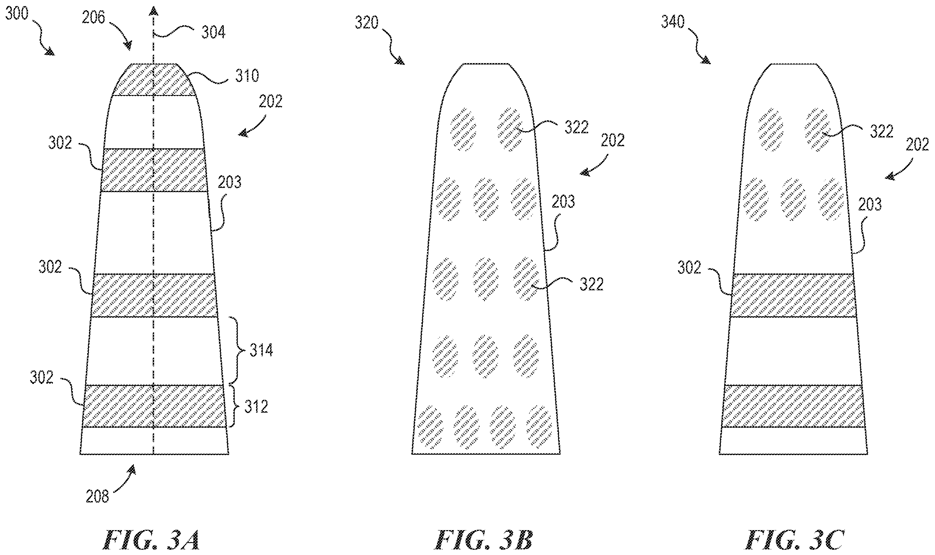

Fig. 3A illustrates an example 300 of an electrode arrangement on and/or embedded within the housing 203. In some embodiments, a series of annular conductive rings 302 are positioned on the exterior surface of the housing 203 and spaced apart along a longitudinal axis 304 of the housing 203 between the first and portion 206 and the second and portion 208. The first end portion 206 can correspond to the end that will abut a portion of the external auditory canal during wear, and the second end portion 208 is configured to be coupled to the electronics module 204 via the first coupling element 210 (not shown). While four conductive rings 302 are illustrated, in other examples the earpiece module 202 can include fewer (e.g., 1-3) or more (e.g., 5-10) conductive rings 302. The contact areas of the electrodes may be formed by depositing or otherwise applying the conductive material to the surface of the housing 203, or by molding the conductive material into depressions in the housing 203. Alternatively, the contacts may be created by forming one or more bulk conductive components on and/or in the housing 203.

The number and spacing of the conductive rings 302 may vary. In one scenario, as many rings as possible are provided so long as the rings do not short one another and are able to obtain reliable high-quality signals that are not influence by signals from neighboring rings. By way of example only, each ring may have a width 312 of between approximately 2-5 mm and the rings may have a spacing 314 of at least approximately 2-5 mm. In another scenario, the rings and/or other conductive element(s) have thinner widths (e.g., no more than 1-2 mm) and spacings (e.g., no more than 1-2 mm apart) to ensure that a sufficient number of rings make contact with different parts of the ear canal and/or provide a minimum signal to noise ratio (e.g., 10 dB, 20 dB or more or less). If certain elements do not provide signals of selected quality, the data received from those elements may be discarded by the on-board processing system of the electronics module or by a remote off-board processing system.

In other examples, any or all of the contacts may have other shapes so long as the contacts circumscribe all or at least a portion of the outer surface of the housing or otherwise provide sufficient signal coverage. Fig. 3B illustrates another example 320 where a series of dots or other shapes of conductive material 322 are distributed along the exterior surface of the housing 203. Fig. 3C illustrates a further example 340 with a combination of rings 302 of conductive material and dots 322 of conductive material. These and other electrode shapes (such as serpentine, zig-zag, arcuate, hemispherical, semicircular, rectangular or other geometric shapes) may be distributed longitudinally and/or radially along the housing or in any other suitable arrangement.

These electrode configurations may provide an orientation agnostic in-ear sensor arrangement that does not require the earpiece module to have a particular rotational orientation in the ear canal. Nonetheless, the earpiece module may include one or more physical reference features (e.g., hash marks, arrows, dots, etc.) so that the wearer may more easily place it at the same clocking orientation each time it is worn, which can aid repeatability for sensing signals. Regardless of the specific configuration, each contact along the exterior surface of the earpiece module housing is associated with a corresponding electrode connector to provide independent sensed signals to the electronics module.

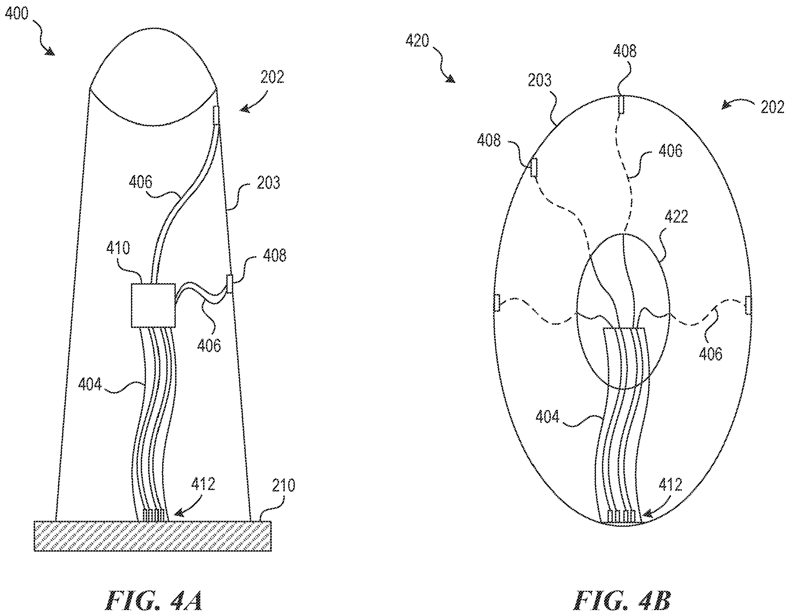

By way of example, Fig. 4A illustrates a partial see-through view 400 of the housing 203 having a flexible printed circuit (FPC) 404 mounted therein. As noted above, the housing 203 comprises a foam or otherwise compliant material (e.g., compressible and/or deformable material) that can be shaped for insertion into the ear canal and then automatically expand to contact the ear canal at multiple points. The FPC 404 includes conductive wires 406 electrically coupled to corresponding contacts/electrodes 408 arranged along the outer surface of the housing 203. In one scenario as shown, the conductive wires 406 extend within the housing 203 and branch off from a common section 410.

Signals detected by the electrodes 408 are passed via the FPC 404 to the electronics module 204 for analysis or other processing. This may be done via one or more contacts 412 that are connected to the first coupling element 210. The conductive wires should be arranged to minimize interference (e.g., cross-coupling) with neighboring wires. Some frequencies of interest may be very low (e.g., below 50 Hz), which minimizes crosstalk. Nonetheless, a ground plane may be incorporated into the FPC 404 so that the conductive wires act as transmission lines instead of unshielded wires. In one scenario, there may only be a single electrode. In this case, the conductive wire may connect anywhere around the circumference of the first coupling element. In other scenarios, two or more electrodes may be employed. In these cases, different regions of the first coupling element may be electrically isolated and connected to corresponding electrodes. For instance, there may be a thin interstitial flexible circuit compressed between the first coupling element and a like flexible circuit (possibly with pogo pins, leaf springs, bumps, etc.) on the side of the second coupling element. The flexible circuits would be patterned with concentric rings or other shapes, each aligned with its partner on the other flexible circuit. A center post/nub may be required to provide alignment between the first and second coupling elements. The on-board signal processor may be configured to clock signals sequentially or in another pattern to gain electrical passthrough for the different electrodes.

Fig. 4B illustrates a partial cutaway end view 420 of the housing 203 and the FPC 404. This view is from the end which will abut a portion of the external auditory canal during use. The wires 406 (shown in dashed lines) extend at least partly through the housing 203 and terminate at the electrodes 408.

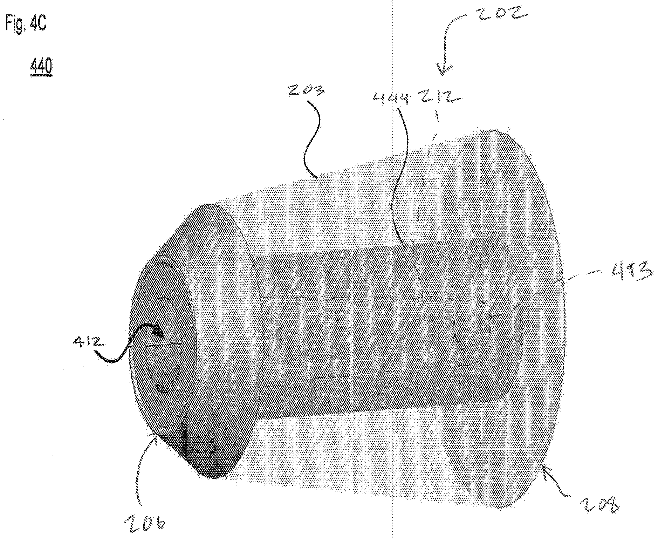

Fig. 4C also shows the through-hole 212 extending through the housing 203 from an opening 412 at the first end portion 206 to an opening 413 (shown in dashed lines) at the second end portion 208. The through-hole 212 provides an acoustic path from the electronics module 204 to the ear canal. To achieve this, the interior of the housing 203 includes a stiffening tube 444 extending generally along the longitudinal axis for sound to pass through. The through-hole 212 is defined by the lumen of the stiffening tube 444, which may be generally cylindrical and extend completely or at least partially through the length of the housing 203. In one example, the stiffening tube 444 may comprise a non-collapsible rigid or semi-rigid material (e.g., plastic) that is inserted into or fabricated as part of the housing 203. The stiffening tube 444 prevents pinching or crimping of the foam or other complaint material at the exterior of the housing 203. This allows the wearer to more efficiently receive acoustic energy without appreciable distortion (e.g., without cutting off or attenuating higher frequencies beyond 10-15 kHz) or reduction in volume. While only one through-hole 212 and stiffening tube 444 are shown in this example, multiple through-holes and/or stiffening tubes may be employed along the length of the housing 203.

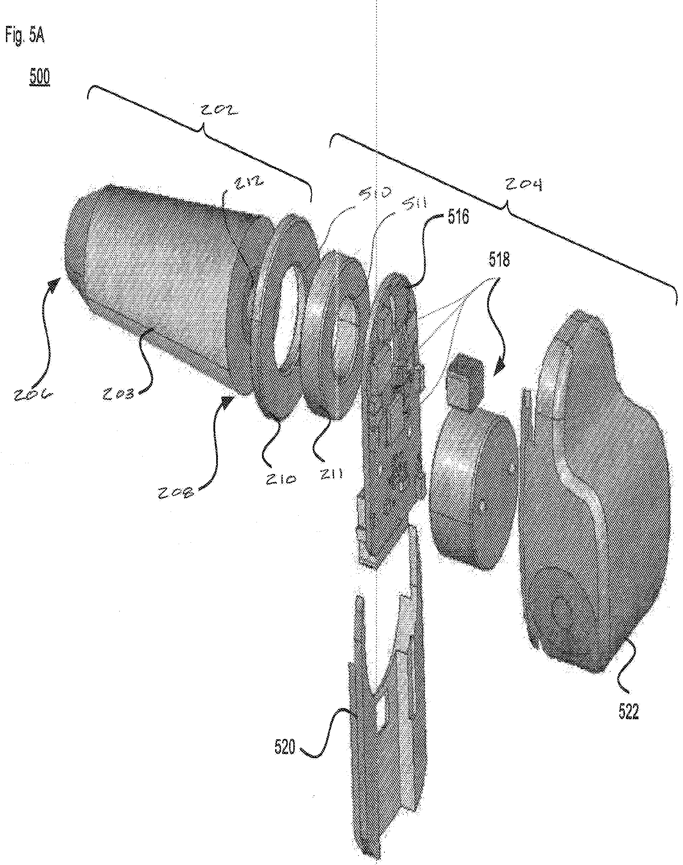

Fig. 5A illustrates an exploded view 500 of the earpiece module 202 and the electronics module 204. As seen in this view, the earpiece module 202 includes the housing 203 having the first end portion 206, the second end portion 208, and the first coupling element 210 at the second end portion 208. In some embodiments, the first coupling element 210 comprises an annular plate having an opening 510 configured to be aligned with the through-hole 212 of the earpiece module 202. In this example, the second coupling element 211 of the electronics module 204 includes a ring magnet having an opening 511 aligned with the opening 510 of the first coupling element 210. The electronics modules 204 can further include a circuit board 516, circuit components 518 coupled to the circuit board 516, a first housing element 520, and a second housing element 522. The circuit components 518 may include, for instance, a speaker, a microphone, a battery, a communication module including wireless and/or wired connections, one or more sensors (e.g., a PPG sensor, a temperature sensor, an accelerometer, an EEG sensor, etc.), one or more indicators (e.g., LEDs), a memory device, as well as an on-board processor such as a controller, CPU or ASIC-based processor. The circuit board 516 may be, by way of example, a double sided, multi-layer printed circuit board, and the circuit components 518 may be surface mounted to both sides of the board.

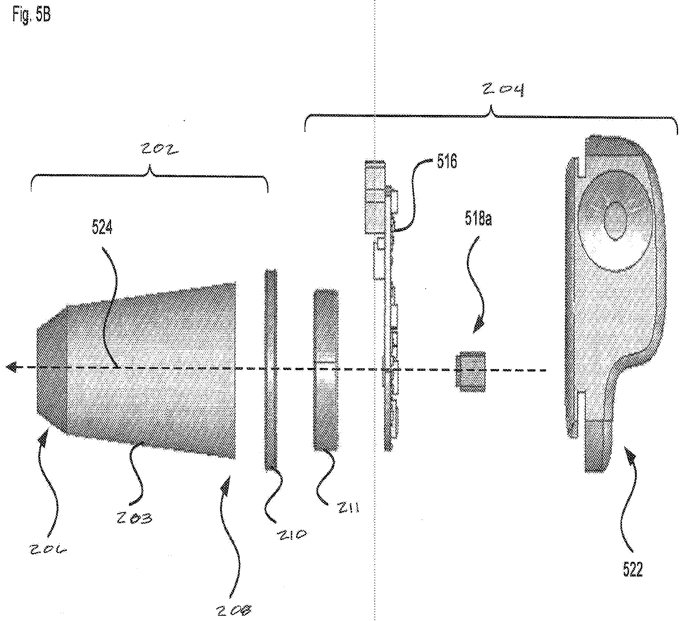

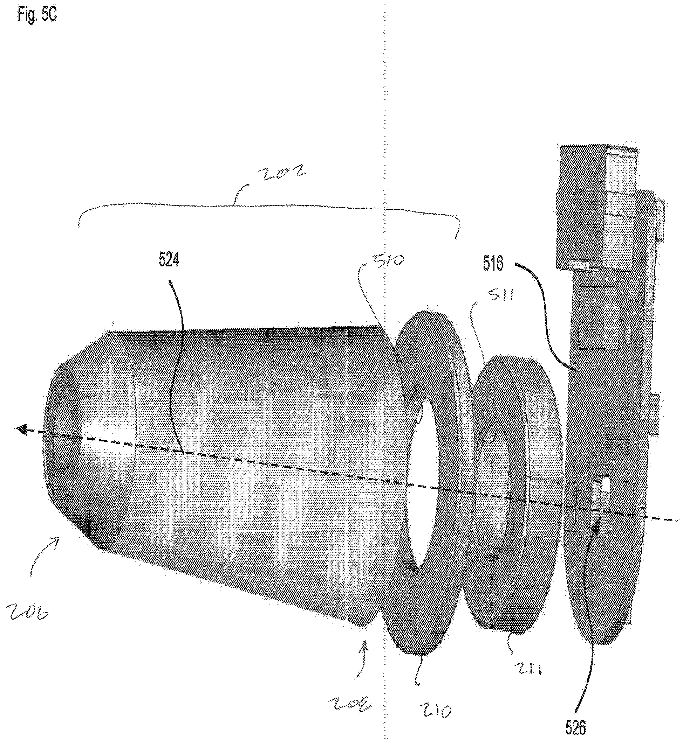

Fig. 5B illustrates a side view omitting one of the larger circuit components 518 and the first housing element 520 for clarity. As seen in this view, the housing 203, the first coupling element 210 (e.g., a metal plate) and the second coupling element 211 (e.g., a ring magnet) are aligned along a longitudinal axis 524. And as seen in the view of Fig. 5C, the circuit board 516 may include an opening 526 aligned with the longitudinal axis 524. In one example, a speaker 518a (Fig. 5A) is mountable on the circuit board 516 about the opening 526 (Fig. 5B) so that the speaker is facing toward the earpiece module 202 and is aligned along the longitudinal axis 524. A microphone may be surface mounted to the side of the circuit board 516 facing the housing 203 so that it directly receives sounds via the through-hole passing through the housing 203.

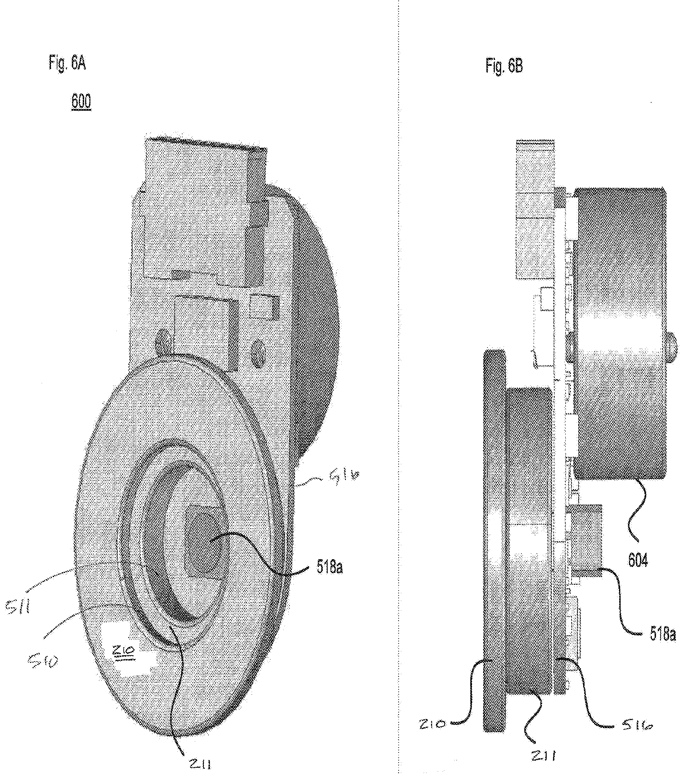

Figs. 6A and 6B illustrate perspective and side views of a portion 600 of the auricular sensing system 200 with the earpiece module 202 omitted for clarity. As shown, the first coupling element 210 is magnetically bonded to the second coupling element 211. In one example, second coupling element 211 is a ring magnet, such as a Neodymium magnet, that is itself electrically connected to a pair of elevated pads (not shown) on the circuit board 516 to complete the electrical path between the electrode(s) of the earpiece module 202 and the circuit board 516. As noted above, different regions of the first coupling element 210 may be electrically isolated and connected to corresponding electrodes, and such regions of the first coupling element 210 may be aligned with a corresponding part along the second coupling element 211. In one example, the second coupling element 211 defined by the ring magnet may be attached to the circuit board pads with a conductive and compliant adhesive to avoid damage in the circuit board during assembly due to reflow temperatures that may exceed the Curie temperature for the magnetic material.

As seen in the perspective view of Fig. 6A, the speaker 518a is arranged in an opening of the circuit board 516 so that is centrally aligned with the openings 510, 511 of the first and second coupling elements 210, 211, respectively. In some embodiments, a digital micro-electromechanical systems (MEMS) microphone may be located along the circuit board 516 adjacent to the speaker opening 526 (Fig. 5B). In this case, the MEMS microphone may be arranged to direct a port downward through the second coupling element 211 into the acoustic tube of the housing 203 formed by the non-collapsible rigid or semi-rigid material. The side view of Fig. 6B also illustrates that a battery module 604 may be affixed to the side of the circuit board 516 opposite from the second coupling element 211.

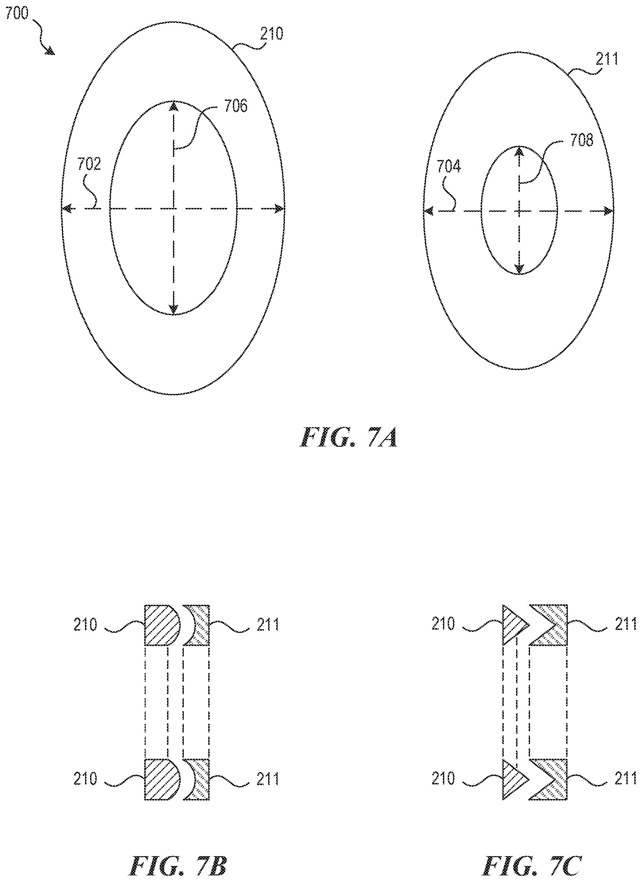

In these examples, both the first and second coupling elements 210, 211 are shown as being annular. Fig. 7 illustrates the first and second coupling elements 210, 211 side by side. As shown, the first coupling element 210 may have a larger outer diameter 702 than the outer diameter 704 of the second coupling element 211. Similarly, in this example the first coupling element 210 may have a larger inner diameter 706 than the inner diameter 708 of the second coupling element 211. In other examples, the inner and/or outer diameters of the first and second coupling elements 210, 211 may be different. For instance, the first coupling element 210 may have a smaller inner or outer diameter than the second coupling 211. The relative inner or outer diameters can vary so long as there is sufficient contact area to pass electrical signals without distortion or clipping exceeding some threshold (e.g., 5-10% signal distortion or clipping), or an error rate below a baseline (e.g., .01%)

The first coupling element 210 can include a ferromagnetic material and be electrically conductive. In one example, the first coupling element 210 may be plated or otherwise coated with a conductive material (e.g., gold). As noted above, the second coupling element 211 may comprise Neodymium, although other magnetic materials may be employed. Like the first coupling element 210, the second coupling element 211 is electrically conductive so that signals obtained by the sensor(s) of the earpiece module 202 can be passed to the processing system of the electronics module 204. The electrical connection should be maintained even when the first coupling element 210 is rotated or translated relative to the second coupling element 211, for instance if the electronics module 204 is repositioned along the concha.

In one scenario, the first coupling element 210 may have a washer-type shape that is flat on both sides. In other scenarios one or both surfaces of the first coupling element 210 may be curved or have some other shape. For instance, referring to Figs. 7B and 7C, the magnet-facing side of the first coupling element 210 may be rounded (Fig. 7B) or angled (Fig. 7C) to mate with an opposing rounded (Fig. 7B) or angled (Fig. 7C) surface of the second coupling element 211. In yet another scenario, a plastic layer may be disposed between the first and second coupling elements 210, 211, with a spring-loaded compression pogo pin, such as in a bahoma-type configuration. And in a further scenario, a spherical or hemispherical shape may be employed for the electrical/magnetic connection. By way of example, a cup-shaped receptacle may be arranged on the first coupling element 210 and/or the second coupling element 211 to receive a corresponding hemispherical projection. This can be used to provide a single electrical path, or several electrical in the manner described above.

### EXAMPLE SYSTEMS

As the one or more sensors of the earpiece module and any sensors in the electronics module gather sensor data, that information is stored and can be processed by a processing system. The stored or processed data may then be downloaded to a remote processing system for further evaluation and analysis.

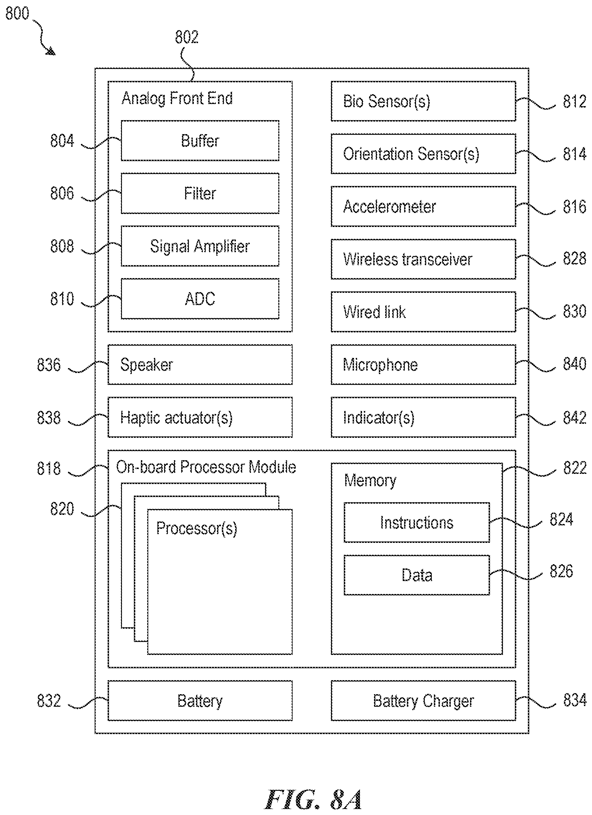

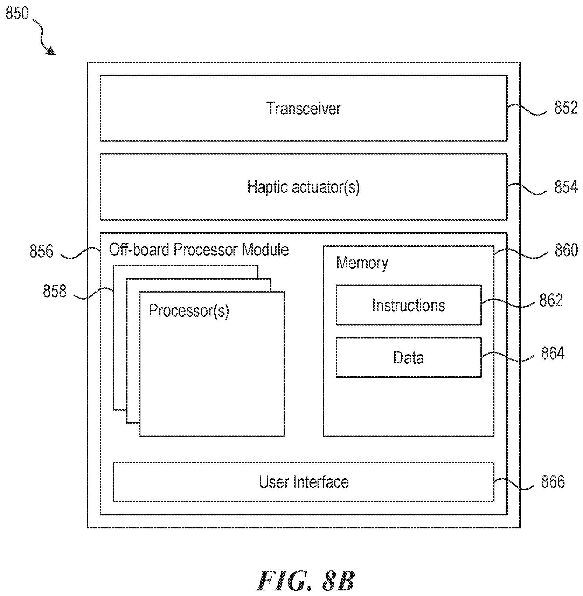

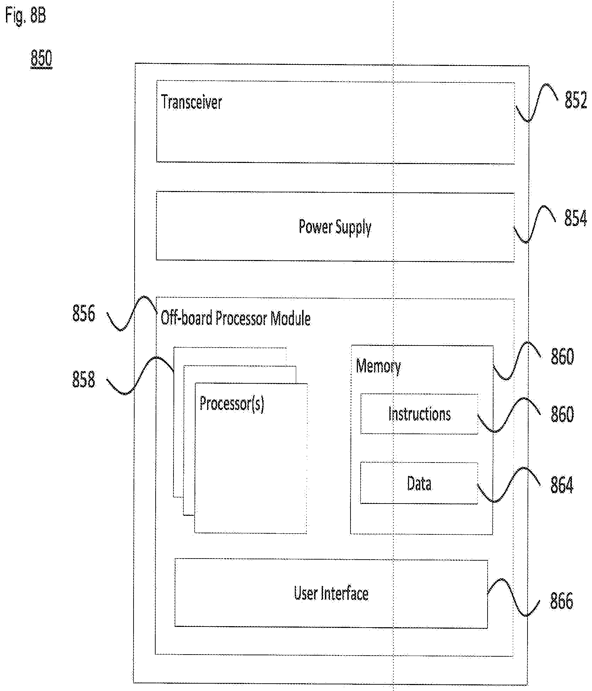

Fig. 8A illustrates one example of an on-board processing system 800 and Fig. 8B illustrates one example of a remote processing system 850. In this example, the signals from the earpiece module electrodes 302, 322, 408 (Figs. 3A-3C and 4A-4B) may be received by an analog front end (AFE) 802, which is in electrical contact with the second coupling element. The AFE 802 may provide one or more of signal buffering via buffer 804, filtering via filter(s) 806, signal amplification by amplifier 808, and/or analog to digital conversion by analog to digital converter (ADC) 810.

The processing system 800 may also receive biometric and other information from additional biosensors 812 of the electronics module, such as sensors that measure temperature, heart rate, EDA / galvanic skin response, blood oxygen levels, glucose levels and/or other physiological parameters. Other sensors may include one or more orientation sensors 814 and an accelerometer 616. Some or all the information from these other sensors may also be processed by AFE 802.

The processing system 800 may analyze the obtained data with an on-board processor module 818, which includes one or more processors 820 as well as memory 822 that stores instructions 824 and data 826 that may be executed or otherwise used by the processor(s) 820. The one or more processors 820 may be, e.g., a controller or CPU. Alternatively, the one or more processors 820 may be a dedicated device such as an ASIC, FPGA or other hardware-based device. By way of example, the device may be a 32-bit or 64-bit RISC multi-core processor module. The memory 822 may be of any type capable of storing information accessible by the processor(s) in a non-transitory manner, such as solid-state flash memory or the like.

The instructions 824 may be any set of instructions to be executed directly (such as machine code) or indirectly (such as scripts) by the processor(s). For example, the instructions may be stored as computing device code in the non-transitory memory. In that regard, the terms "instructions" and "programs" may be used interchangeably herein. The instructions may be stored in object code format for direct processing by the processor(s), or in any other computing device language including scripts or collections of independent source code modules that are interpreted on demand or compiled in advance. The data 826 may be retrieved, stored or modified by one or more processors in accordance with the instructions 824. As an example, data 826 may include heuristics to be used when calibrating or evaluating electrode response characteristics.

Alternatively, or in addition to on-board signal analysis, the processing system may transmit the obtained data to remote processing system 850. This may be done, for instance, via a wireless transceiver 828 or a wired link 830, such as a micro USB or other communications path. In the former case, the wireless transceiver 828 may communicate with the remote processing system 850 via Bluetooth^{™}, Bluetooth^{™} LE, near field communication (NFC) or some other wireless communication method. In the former case, the antenna for the wireless transceiver may be a monopole antenna configured for operation in the 2.4 GHz band and may be located along an inner top surface of the electronics module. In the latter case, a flexible printed circuit, USB cable or other wired connection may be made with coupling 216 of Fig. 2D, so that processing system 850 that can receive and/or process the obtained bio-signals and other information from the earpiece module.

The process system 800 also includes a battery 832 to power the components of the system. It may also include a battery charger 834. The battery charger may be contactless or plugged into an external power source to charge the battery, for instance when the micro USB cable is connected to the remote processing system 850. In one example, a single cell Lithium polymer battery (e.g., on the order of 45-90 mAH) may be soldered directly to the circuit board or removably affixed to a battery receptacle. The battery cell(s) may be protected from overvoltage and current, as well as over discharge by protection circuitry located on a battery protection board (not shown).

The state of charge can be monitored by system software, for instance which can measure the battery voltage through an ADC pin. In one scenario, some or all of the components of the earpiece module may run off of a 3.3V supply produced by a small low dropout (LDO) regulator. Other components, such as a PPG sensor chip mounted to the circuit board, may also connect directly to the battery but operate at a higher (or lower) voltage. Such components may have their own internal LDO regulator. The microphone may be directly powered by a general purpose I/O (GPIO) system on chip (SOC) unit that is coupled to the battery. In this scenario, the system may be able to enter an idle power mode of just a few microamps. The Lithium polymer cell may be charged by a linear charger, for instance when connected to the remote processing system or an off-board charger device.

In one example, the PPG sensor is configured to operate in reflectance mode to measure pulse rate. The general mechanism is to measure the light reflected by tissue and blood at particular wavelengths generated by the sensor's internal LEDs. For instance, the sensor can control three internal LED intensities (e.g., green, red and IR), and measure current flowing in a photodiode. The sensor may sequence the LED illumination and photodiode measurements to characterize reflected light levels when each LED is lit, as well as ambient light levels when they are not glowing. In addition to measuring base heart rate, the sensor information may be processed to produce heart rate variability (HRV) and oxygen saturation (SP02), as well as respiration rate. In one scenario, the PPG sensor is mounted along the electronics module so that the optical surface of the sensor is in direct contact with the wearer's skin. Ideally, as much light as possible that reaches the photodiode passes through tissue and has the opportunity to be intensity modulated by pulsatile blood flow. The PPG subsystem may operate at between 50-250 samples per second. A range of signal processing techniques (filters, correlators, FFT/DFTs) may be employed by the processing system to extract useful information from a weak PPG signal that can be corrupted by motion artifacts.

Other biosensors can include temperature sensors, EEG sensors and/or galvanic skin response sensors. In one example, the ADC 810 may be able to sense temperature, although its accuracy may vary due to self-heating and indirect contact with the ear. In another example, an infrared non-contact temperature sensor may be aimed along longitudinal axis 524, through the plug. Alternatively, or additionally, a sensor that measures the temperature within the electronics module, which can closely correlate with the wearer's temperature.

As discussed above with regard to Figs. 5A-5C, in one example a speaker 836 may be incorporated into the system 800. The speaker 836 is operatively coupled to the on-board processor module 818 to provide sound to the inner portions of the canal. The module 818 may actuate the speaker 836 to supplement (augment) sounds passed through the ear canal, or to generate different sounds such as audible cues (e.g., tones, beeps, clicks, chirps, white noise, etc.) to provide information, give notifications, give experimental stimulus, or give aural feedback to the wearer. The speaker may be able to produce audio signals across the full range of human hearing or across a more limited range. Alternatively or additionally, one or more haptic actuators 838 may be employed to give haptic feedback or other physical sensations to the wearer.

A microphone 840 may be positioned along the circuit board or otherwise placed within the electronics module so that it is able to directly receive audio signals from the acoustical path formed by the stiffening tube. In one example, the microphone is able to output a pulse delay modulation (PDM) data stream on the order of 500 Kbps to 1500 Kbps. This data stream may be sampled and converted into a time series on the order of 10-20 kHz for internal processing. The signal may not be stored or passed along in its raw form; rather it can be processed to extract a low-frequency amplitude envelope (sound pressure level) that is represented in a very low samples/second data rate on the order of 10-30. The sound pressure level usable dynamic range of this module may be between 20dB/A to 120dB/A, or more or less.

In addition, one or more indicators 842, such as LEDs, may be positioned on the circuit board or elsewhere within or along the electronics module. By way of example, one LED may be used as user indicator. When mounted on the circuit board, the LED can have a light pipe up to the enclosure surface of the electronics module. Another LED may be configured for an optical sync reference when communicating with a remote off-board system.

The processing system 800 may be incorporated into or mounted on the electronics module 204 (Figs. 2A-2G) as a monolithic integrated circuit or as a set of discrete components. For instance, the on-board processing system could be integrated as a system on chip (SOC) module mounted to the circuit board 516 (Figs. 5A-5B). By way of example only, the AFE 802, sensors 812, 814 and 816, the battery 832, the battery charger 834, the speaker 836, the haptic actuator(s) 838, microphone 840 and/or indicators 842 need not be co-located with the on-board processor module 818. Rather, the individual components can be distributed within or along different parts of the housing for the electronics module 204. In one scenario, the circuit board may be medium density double sided SMT multilayer circuit board 516. The components mounted on either side of the circuit board may be constrained to be 1.0 mm high or less. In one scenario, the circuit board itself is a four-layer .8 mm thick board.

According to one aspect of the technology, a given earpiece module combines the data collection from the various sensors with on-board processing and data link/storage elements. However, in other scenarios the earpiece module may primarily gather sensor data and transmit it off-board for processing.

Fig. 8B schematically shows a remote processing system 850 including a transceiver 852 configured to communicate with one or both of wireless transceiver 828 and wired link 830. The system 850 also includes a power supply 854, which may include batteries and/or a connection for an outlet or the like. The power supply 854 may be able to recharge the battery of the earpiece module. The information received from the on-board processing system 800, whether raw or unprocessed, is passed from the transceiver 852 to the off-board processor module 856.

The off-board processor module 856 is configured to analyze the obtained data with one or more processors 858 and memory 860 that stores instructions 862 and data 864 that may be executed or otherwise used by the processor(s) 858, in a manner similar to described above. The one or more processors 858 may be, e.g., a controller or CPU. Alternatively, the one or more processors 858 may be a dedicated device such as a DSP, an ASIC, FPGA or other hardware-based device. The memory 860 may be of any type capable of storing information accessible by the processor(s) in a non-transitory manner, such as solid state flash memory, hard disc, optical medium or the like. The off-board processor module 856 may also include a user interface subsystem 866, which may be used to present information regarding the processed data to the wearer, a technician, doctor or other authorized user.

As noted above, it may be desirable for the modular auricular sensing system to be worn for extended periods of time. This can provide a wealth of information that can be used for different purposes. For instance, temperature, including heat exchange between two points and heat expulsion, can be evaluated. The temperature may vary depending on whether the wearer is sleeping, sitting, moving, etc. Different kinds of brain activity can be measured and compared to what else is going on with the body.

At the time of use, the sensing system may be assembled by magnetically connecting the earpiece module to the electronics module via engaging the first coupling element with the second coupling element. Different earpiece modules may be available. In the case of magnetic coupling, the earpiece module may be changed quickly, for instance to change the size of the earpiece module, to change the electrode configuration, for repair or cleaning, etc. Similarly, different electronics modules may be utilized, for instance to change to a different set of sensors, recharge the battery, or download data to the remote processing system.

The compliant outer portion of the housing of the earpiece module will be compressed and inserted deeply into the ear canal, providing a broad and relatively large contact surface for the electrode(s). In one scenario, the earpiece module can be held in place as the foam or other compliant material expands, and then the electronics module can be attached to the earpiece module (e.g., by magnetic attraction). Bias and reference conductive electrode patches (see, e.g., 214 in Figs. 2A-2C) on the body of the electronics module 204 can be positioned by the wearer (e.g., by rotating and/or sliding the magnetic attachment point) to complete the electrical connections. Different electronics modules may be swapped one for another while the earpiece module remains in the ear canal. In another scenario, the earpiece module and electronics module may be coupled together before inserting the earpiece module into the ear canal.

The present invention is defined by the appended claims
Unless otherwise stated, the foregoing alternative examples are not mutually exclusive, but may be implemented in various combinations to achieve unique advantages. As these and other variations and combinations of the features discussed above can be utilized without departing from the subject matter defined by the claims, the foregoing description of the embodiments should be taken by way of illustration rather than by way of limitation of the subject matter defined by the claims. In addition, the provision of the examples described herein, as well as clauses phrased as "such as," "including" and the like, should not be interpreted as limiting the subject matter of the claims to the specific examples; rather, the examples are intended to illustrate only one of many possible embodiments. Further, the same reference numbers in different drawings can identify the same or similar elements. The processes or other operations may be performed in a different order or simultaneously, unless expressly indicated otherwise herein.

## Claims

1. A modular auricular sensing system (200), comprising:
an earpiece module (202) having a housing (203), a first coupling element (210) and at least one electrode (322), the housing (203) including a first end portion (206) configured for at least partial insertion into an ear canal and a second end portion (208) opposite the first end portion (206);
wherein the first coupling element (210) is secured to the second end portion (208) of the housing (203), and the electrode (322) is arranged along the housing (203) between the first end portion (206) and the second end portion (208); and
an electronics module (204) having a second coupling element (211) at a first side thereof, the second coupling element (211) being releasably attached to the first coupling element (210);
wherein the first coupling element (210) and the second coupling element (211) facilitate electrical connection between the earpiece module (202) and the electronics module (204) to transmit electrical signals from the electrode (322) to the electronics module (204); and
**characterised in that**:
the earpiece module (202) has a stiffening tube (444) extending along a longitudinal axis from the first end portion (206) to the second end portion (208), and wherein the housing (203) of the earpiece module (202) has a through-hole (212) therethrough from the first end portion (206) to the second end portion (208) defined by a lumen of the stiffening tube (444), and wherein the through-hole (212) extends through the stiffening tube (444) providing an acoustic path from the electronics module (204) to the ear canal.

2. The modular auricular sensing system (200) of claim 1, wherein the first coupling element (210) comprises a plate and the second coupling element (211) comprises a magnet.

3. The modular auricular sensing system (200) of claim 2, wherein:
the plate comprises a metal plate and the magnet comprises a ring magnet; and
when coupled together, the ring magnet and the metal plate enables at least one of rotation or translation of the metal plate relative to the ring magnet while maintaining electrical connectivity between the earpiece module (202) and the electronics module (204).

4. The modular auricular sensing system (200) according to any of claims 1-3, wherein the electronics module (204) includes a speaker (518a).

5. The modular auricular sensing system (200) of claim 4, wherein the electronics module (204) includes a microphone (840) acoustically coupled to the earpiece module (202) via the through-hole (212) of the earpiece module (202).

6. The modular auricular sensing system (200) according to any of claims 1-5, wherein the first coupling element (210) and the second coupling element (211) are both annular, optionally wherein the first coupling element (210) has a larger outer diameter than an outer diameter of the second coupling element (211); optionally wherein the first coupling element (210) has a larger inner diameter than an inner diameter of the second coupling element (211).

7. The modular auricular sensing system (200) according to any of claims 1-6, wherein the first coupling element (210) is electrically coupled to the electrode (322) and the second coupling element (211) is electrically coupled to an electronic component of the electronics module (204), and electrical signals are transmitted from the electrode (322) to the electronics module (204) via the first coupling element (210) and the second coupling element (211).

8. The modular auricular sensing system (200) according to any of claims 1-7, wherein the first coupling element (210) comprises a ferromagnetic material and is coated with an electrically conductive material, and/or wherein the second coupling element (211) comprises Neodymium.

9. The modular auricular sensing system (200) according to any of claims 1-8, wherein the first coupling element (210) is washer-shaped having a first side permanently secured to the second end portion (208) of the housing (203); and/or wherein a second side of the first coupling element (210) is curved to mate with a contact surface of the second coupling element (211).

10. The modular auricular sensing system (200) according to any of claims 1-9, wherein the electronics module (204) includes a processor module operatively coupled to the second coupling element (211) to receive the electrical signals and to perform on-board processing of the received electrical signals or to transmit the received electrical signals to a remote processing system (850).

11. The modular auricular sensing system (200) according to any of claims 1-10, wherein the electronics module (204) further includes one or more additional sensors, including at least one of a biosensor, an orientation sensor, or an accelerometer; optionally wherein the one or more additional sensors includes a temperature sensor.

12. The modular auricular sensing system (200) according to any of claims 1-11, further comprising a pair of interstitial flexible circuits compressed between the first coupling element (210) and the second coupling element (211), the pair of interstitial flexible circuits being configured to pass the electrical signals from the first coupling element (210) to the second coupling element (211).

## Patentansprüche

1. Modulares aurikuläres Sensorsystem (200), umfassend:
ein Ohrstückmodul (202), aufweisend ein Gehäuse (203), ein ersten Kupplungselement (210) und mindestens eine Elektrode (322), wobei das Gehäuse (203) einen ersten Endabschnitt (206), der ausgelegt ist, um zumindest teilweise in einen Gehörgang eingefügt zu werden, und einen zweiten Endabschnitt (208), der dem ersten Endabschnitt (206) entgegengesetzt ist, beinhaltet,
wobei das erste Kupplungselement (210) an dem zweiten Endabschnitt (208) des Gehäuses (203) befestigt ist und die Elektrode (322) entlang des Gehäuses (203) zwischen dem ersten Endabschnitt (206) und dem zweiten Endabschnitt (208) angeordnet ist, und
ein Elektronikmodul (204), aufweisend an einer ersten Seite ein zweites Kupplungselement (211), wobei das zweite Kupplungselement (211) lösbar an dem ersten Kupplungselement (210) angebracht ist,
wobei das erste Kupplungselement (210) und das zweite Kupplungselement (211) die elektrische Verbindung zwischen dem Ohrstückmodul (202) und dem Elektronikmodul (204) erleichtern, um elektrische Signale von der Elektrode (322) zu dem Elektronikmodul (204) zu übertragen, und
**dadurch gekennzeichnet ist, dass**:
das Ohrstückmodul (202) einen Versteifungsschlauch (444) aufweist, der sich entlang einer Längsachse von dem ersten Endabschnitt (206) zu dem zweiten Endabschnitt (208) erstreckt, und wobei das Gehäuse (203) des Ohrstückmoduls (202) ein dadurch führendes Durchgangsloch (212) von dem ersten Endabschnitt (206) zu dem zweiten Endabschnitt (208) aufweist, definiert durch ein Lumen des Versteifungsschlauchs (444), und wobei sich das Durchgangsloch (212) durch den Versteifungsschlauch (444) erstreckt und einen akustischen Weg von dem Elektronikmodul (204) zu dem Hörgang bereitstellt.

2. Modulares aurikuläres Sensorsystem (200) nach Anspruch 1, wobei das erste Kupplungselement (210) eine Platte umfasst und das zweite Kupplungselement (211) einen Magneten umfasst.

3. Modulares aurikuläres Sensorsystem (200) nach Anspruch 2, wobei
die Platte eine Metallplatte umfasst und der Magnet einen Ringmagneten umfasst und
der Ringmagnet und die Metallplatte, wenn sie miteinander gekuppelt sind, mindestens entweder eine Drehung oder Verschiebung der Metallplatte relativ zum Ringmagneten ermöglichen, während die elektrische Verbindungsfähigkeit zwischen dem Ohrstückmodul (202) und dem Elektronikmodul (204) aufrechterhalten wird.

4. Modulares aurikuläres Sensorsystem (200) nach einem der Ansprüche 1-3, wobei das Elektronikmodul (204) einen Lautsprecher (518a) beinhaltet.

5. Modulares aurikuläres Sensorsystem (200) nach Anspruch 4, wobei das Elektronikmodul (204) ein Mikrofon (840) beinhaltet, das über das Durchgangsloch (212) des Ohrstückmoduls (202) akustisch mit dem Ohrstückmodul (202) gekuppelt ist.

6. Modulares aurikuläres Sensorsystem (200) nach einem der Ansprüche 1-5, wobei das erste Kupplungselement (210) und das zweite Kupplungselement (211) beide ringförmig sind, wobei das erste Kupplungselement (210) optional einen größeren Außendurchmesser als ein Außendurchmesser des zweiten Kupplungselements (211) aufweist, wobei das erste Kupplungselement (210) optional einen größeren Innendurchmesser als ein Innendurchmesser des zweiten Kupplungselements (211) aufweist.

7. Modulares aurikuläres Sensorsystem (200) nach einem der Ansprüche 1-6, wobei das erste Kupplungselement (210) elektrisch mit der Elektrode (322) gekuppelt ist und das zweite Kupplungselement (211) elektrisch mit einer elektronischen Komponente des Elektronikmoduls (204) gekuppelt ist und elektrische Signale von der Elektrode (322) über das erste Kupplungselement (210) und das zweite Kupplungselement (211) an das Elektronikmodul (204) übertragen werden.

8. Modulares aurikuläres Sensorsystem (200) nach einem der Ansprüche 1-7, wobei das erste Kupplungselement (210) ein ferromagnetisches Material umfasst und mit einem elektrisch leitfähigen Material beschichtet ist, und/oder wobei das zweite Kupplungselement (211) Neodym umfasst.

9. Modulares aurikuläres Sensorsystem (200) nach einem der Ansprüche 1-8, wobei das erste Kupplungselement (210) scheibenförmig ist und eine erste Seite aufweist, die dauerhaft an dem zweiten Endabschnitt (208) des Gehäuses (203) befestigt ist, und/oder wobei eine zweite Seite des ersten Kupplungselements (210) gekrümmt ist, um mit einer Kontaktoberfläche des zweiten Kupplungselements (211) zusammenzupassen.

10. Modulares aurikuläres Sensorsystem (200) nach einem der Ansprüche 1-9, wobei das Elektronikmodul (204) ein Prozessormodul beinhaltet, das betriebswirksam mit dem zweiten Kupplungselement (211) gekuppelt ist, um die elektrischen Signale zu empfangen und eine geräteseitige Verarbeitung der empfangenen elektrischen Signale durchzuführen oder die empfangenen elektrischen Signale an ein entferntes Verarbeitungssystem (850) zu übertragen.

11. Modulares aurikuläres Sensorsystem (200) nach einem der Ansprüche 1-10, wobei das Elektronikmodul (204) ferner einen oder mehrere zusätzliche Sensoren beinhaltet, einschließlich mindestens eines Biosensors, eines Orientierungssensors oder eines Beschleunigungsmessers, wobei der eine oder die mehreren zusätzlichen Sensoren optional einen Temperatursensor beinhalten.

12. Modulares aurikuläres Sensorsystem (200) nach einem der Ansprüche 1-11, ferner umfassend ein Paar interstitieller flexibler Leiterbahnen, die zwischen dem ersten Kupplungselement (210) und dem zweiten Kupplungselement (211) komprimiert sind, wobei das Paar interstitieller flexibler Leiterbahnen ausgelegt ist, um die elektrischen Signale von dem ersten Kupplungselement (210) zu dem zweiten Kupplungselement (211) weiterzuleiten.

## Revendications

1. Système modulaire de détection auriculaire (200), comprenant :
un module d'écouteur (202) comportant un logement (203), un premier élément de couplage (210) et au moins une électrode (322), le logement (203) comportant une première partie d'extrémité (206) configurée pour une insertion au moins partielle dans un conduit auditif et une seconde partie d'extrémité (208) opposée à la première partie d'extrémité (206) ;
le premier élément de couplage (210) étant fixé à la seconde partie d'extrémité (208) du logement (203), et l'électrode (322) étant disposée le long du logement (203) entre la première partie d'extrémité (206) et la seconde partie d'extrémité (208) ; et
un module électronique (204) comportant un second élément de couplage (211) sur un premier côté de celui-ci, le second élément de couplage (211) étant fixé de manière amovible au premier élément de couplage (210) ;
le premier élément de couplage (210) et le second élément de couplage (211) facilitant la connexion électrique entre le module d'écouteur (202) et le module électronique (204) pour transmettre des signaux électriques de l'électrode (322) au module électronique (204) ; et
**caractérisé en ce que** :
le module d'écouteur (202) comporte un tube raidisseur (444) s'étendant le long d'un axe longitudinal de la première partie d'extrémité (206) à la seconde partie d'extrémité (208), et le logement (203) du module d'écouteur (202) comportant un trou traversant (212), allant de la première partie d'extrémité (206) à la seconde partie d'extrémité (208), défini par une lumière du tube raidisseur (444), et le trou traversant (212) s'étendant à travers le tube raidisseur (444) fournissant un chemin acoustique du module électronique (204) au canal auditif.

2. Système modulaire de détection auriculaire (200) selon la revendication 1, le premier élément de couplage (210) comprenant une plaque et le second élément de couplage (211) comprenant un aimant.

3. Système modulaire de détection auriculaire (200) selon la revendication 2, dans lequel :
la plaque comprend une plaque métallique et l'aimant comprend un anneau magnétique ; et
lorsqu'ils sont accouplés, l'aimant annulaire et la plaque métallique permettent au moins une rotation ou une translation de la plaque métallique par rapport à l'aimant annulaire tout en maintenant la connectivité électrique entre le module d'écouteur (202) et le module électronique (204).

4. Système modulaire de détection auriculaire (200) selon l'une quelconque des revendications 1-3, le module électronique (204) comprenant un haut-parleur (518a).

5. Système modulaire de détection auriculaire (200) selon la revendication 4, le module électronique (204) comprenant un microphone (840) couplé acoustiquement au module d'écouteur (202) via le trou traversant (212) du module d'écouteur (202).

6. Système modulaire de détection auriculaire (200) selon l'une quelconque des revendications 1-5, le premier élément de couplage (210) et le second élément de couplage (211) étant tous deux annulaires, éventuellement le premier élément de couplage (210) ayant un diamètre extérieur supérieur au diamètre extérieur du second élément de couplage (211) ; éventuellement le premier élément de couplage (210) ayant un diamètre intérieur supérieur au diamètre intérieur du second élément de couplage (211).

7. Système modulaire de détection auriculaire (200) selon l'une quelconque des revendications 1-6, le premier élément de couplage (210) étant couplé électriquement à l'électrode (322) et le second élément de couplage (211) étant couplé électriquement à un composant électronique du module électronique (204), et les signaux électriques étant transmis de l'électrode (322) au module électronique (204) via le premier élément de couplage (210) et le second élément de couplage (211).

8. Système modulaire de détection auriculaire (200) selon l'une quelconque des revendications 1-7, le premier élément de couplage (210) comprenant un matériau ferromagnétique et étant recouvert d'un matériau électroconducteur, et/ou le second élément de couplage (211) comprenant du néodyme.

9. Système modulaire de détection auriculaire (200) selon l'une quelconque des revendications 1-8, le premier élément de couplage (210) étant en forme de rondelle ayant un premier côté fixé de façon permanente à la seconde partie d'extrémité (208) du logement (203) ; et/ou un second côté du premier élément de couplage (210) étant incurvé pour s'adapter à une surface de contact du second élément de couplage (211).

10. Système modulaire de détection auriculaire (200) selon l'une quelconque des revendications 1-9, le module électronique (204) comprenant un module de processeur couplé de manière fonctionnelle au second élément de couplage (211) pour recevoir les signaux électriques et effectuer un traitement embarqué des signaux électriques reçus ou pour transmettre les signaux électriques reçus à un système de traitement à distance (850).

11. Système modulaire de détection auriculaire (200) selon l'une quelconque des revendications 1-10, le module électronique (204) comprenant en outre un ou plusieurs capteurs supplémentaires, dont au moins un biocapteur, un capteur d'orientation ou un accéléromètre ; éventuellement, le ou les capteurs supplémentaires comprenant un capteur de température.

12. Système modulaire de détection auriculaire (200) selon l'une quelconque des revendications 1-11, comprenant en outre une paire de circuits flexibles interstitiels comprimés entre le premier élément de couplage (210) et le second élément de couplage (211), la paire de circuits flexibles interstitiels étant configurée pour transmettre les signaux électriques du premier élément de couplage (210) au second élément de couplage (211).
